**Europäisches Patentamt**

**European Patent Office**

**Office européen des brevets**

(19)

(11) Veröffentlichungsnummer: **0 018 561**
**B1**

(12) **EUROPÄISCHE PATENTSCHRIFT**

(45) Veröffentlichungstag der Patentschrift:
**24.09.86**

(21) Anmeldenummer: **80102105.6**

(22) Anmeldetag: **18.04.80**

(51) Int. Cl.⁴: **A 61 K 35/16,** A 61 K 35/50,
A 61 K 37/47, A 61 K 37/475,
A 61 K 37/64

(54) **Verfahren zur Stabilisierung von Blutgerinnungsfaktoren.**

(30) Priorität: **25.04.79 DE 2916711**

(43) Veröffentlichungstag der Anmeldung:
**12.11.80 Patentblatt 80/23**

(45) Bekanntmachung des Hinweises auf die Patenterteilung:
**24.09.86 Patentblatt 86/39**

(84) Benannte Vertragsstaaten:
**AT CH DE FR GB IT LI SE**

(56) Entgegenhaltungen:
**BE-A-877 439**
**DE-A-2 653 534**
**GB-A-941 019**

(73) Patentinhaber: **BEHRINGWERKE AKTIENGESELLSCHAFT, Postfach 1140, D-3550 Marburg/Lahn (DE)**

(72) Erfinder: **Schwinn, Horst, Dr., Stümpelstal 27, D-3550 Marburg/Lahn (DE)**
Erfinder: **Heimburger, Norbert, Dr., Sonnenhang 10, D-3550 Marburg/Lahn (DE)**
Erfinder: **Kumpe, Gerhardt, Aspherfeld 14, D-3552 Wetter (DE)**
Erfinder: **Herchenhan, Bernd, Im Brand 33, D-3570 Kirchhain (DE)**

(74) Vertreter: **Meyer- Dulheuer, Karl- Hermann, Dr., HOECHST Aktiengesellschaft Zentrale Patentabteilung Postfach 80 03 20, D-6230 Frankfurt/Main 80 (DE)**

**Beschreibung**

Die Erfindung betrifft ein Verfahren zur Herstellung von Blutgerinnungsfaktoren, die in die Blutgerinnung fördernd oder ihr entgegenwirkend eingreifen, das sind Gerinnungs- und Fibrinolyse-Faktoren. Diese werden in diesem Verfahren einer Wärmebehandlung unterworfen, um bei ihrer Anwendung eine Hepatitis-Übertragung auszuschließen. Ein besonderes Merkmal von nach diesem Verfahren hergestellten praparationen ist, daß sie praktisch frei von gerinnbarem Fibrinogen sind.

Die Blutgerinnung ist ein komplexer, in Stufen ablaufender Vorgang, der durch verschiedene physiologische wie pathologische Ursachen ausgelöst wird und dessen Ablauf von etwa 20 fördernden und hemmenden Faktoren abhängt. Durch Verminderung oder Vermehrung dieser Blutgerinnungsfaktoren treten Störungen der Blutgerinnung auf, die sich teilweise als Krankheiten manifestieren.

So führt z.B. eine Erkrankung der Leber mit verminderter Syntheseleistung dieses Organs zu einem Abfall des Plasma-Prothrombin (Faktor II)-Spiegels, ein Vorgang, der zu spontanen, lebensbedrohenden Blutungen führen kann. Prothrombin-Konzentrate sind hier das Mittel der Wahl für eine sofort wirkende Therapie.

Die Hämophilie A ist durch eine Verminderung des Faktors VIII der Blutgerinnung bedingt und durch Blutungen, besonders in die Gelenke und die Muskulatur, charakterisiert. Es hat sich in den letzten Jahren gezeigt, daß die Prognose der an dieser Hämophilie Erkrankten durch substituierende Therapie von Faktor VIII enthaltenden Praparaten wesentlich gebessert werden kann.

Der Blutgerinnungsfaktor XIII greift in die letzte Phase der Blutgerinnung in dem Sinne ein, daß monomeres Fibrin polymerisiert wird. Faktor XIII wird deshalb auch häufig fibrinstabilisierender Faktor genannt. Ein bei Faktor XIII-Mangel gebildetes Fibringerinnsel ist relativ leicht wieder abbaubar mit der Folge einer gestörten Wundheilung. Faktor XIII-Präparationen werden deshalb klinisch an Patienten mit einer kongenitalen oder erworbenen Faktor XIII-Mangelerkrankung appliziert.

Das Antithrombin III wirkt inhibierend auf Thrombin und aktivierten Faktor X (F Xa) sowie andere Serinproteasen in der Reihe der Gerinnungsfaktoren. Es kommt ihm deshalb eine große Bedeutung in der Steuerung der Blutgerinnung zu. Bereits relativ geringe Verminderungen des Antithrombingehaltes im Blut führen zu einer betrachtlichen Erhöhung des Thrombose-Risikos.

Das Plasminogen ist das zentrale Protein der fibrinolytischen Aktivität des Plasmas. Es ist das Zymogen des plasmins, einer Protease mit einer hohen Spezifität fur Fibrin und Fibrinogen. Nach abgeschlossener Thrombusbildung und Einsetzen der Wundheilung wird in einer lokalen Proteolyse das Fibringerinnsel durch Plasmin abgebaut. Plasminogenmängel, wie sie z.B. unter einer Fibrinolysetherapie vorübergehend auftreten, können mit Plasminogenkonzentraten erfolgreich therapiert werden.

Es sind verschiedene Verfahren zur Herstellung von humanen Faktor II-, VIII-, XIII- und Antithrombin III sowie Plasminogenkonzentraten aus Blut, Blutplasma oder Plazenten bekannt.

So kann ein Faktor II-Prapärat nach Thrombosis Diath. Haemorrh. Suppl. 35, 62 (1969) hergestellt werden. Die Herstellung von Faktor VIII-Konzentrat ist beispielsweise nach der sogenannten Methode IV (Blood (1966) 28, 1011) unter Verwendung von Polyethylenglykol möglich.

Faktor XIII kann beispielsweise nach H. Bohn; Blut 25, 235 (1972) aus humanen Plazenten gewonnen werden.

In der JP-A- 5 359 018 ist ein Verfahren zur Herstellung eines Faktor XIII-Konzentrates beschrieben. Zur Inaktivierung von Hepatits-Viren wird darin eine Lösung dieses Faktors in Gegenwart von 10-20 % (W:V) einer neutralen Aminosäure, eines Monosaccharids oder Zuckeralkohols 9-11 Stunden auf 55-65° C erwärmt.

Antithrombin III und Plasminogen können nach Verfahren hergestellt werden, wie sie in Antithrombin, uterine Hämostase, Herz und Blutgerinnung, Verhandlungen der Dt. Arbeitsgemeinschaft für Blutgerinnung, 1971, S. 1-16, DE-AS 22 43 688, Science 170, 1095 (1970) oder DE-A-20 57 401 beschrieben werden.

In einem Verfahren der Albuminherstellung, das ein als hepatitis-sicher angesehenes Produkt ergibt (J. Clin. Invest. (1948) 27, 239), wird die Erhitzung als wesentlich erachtet. Folglich sollten auch Konzentrate der Gerinnungsfaktoren in wäßriger Lösung mindestens 10 Std. auf 60°C erhitzt werden. Eine solche Behandlung von Faktor XIII ist zwar in obigem japanischen Patent schon beschrieben, doch führt sie zu erheblichen Ausbeuteverlusten. Für alle anderen Präparate ist eine Stabilisierung in einem Erhitzungsverfahren bisher unbekannt. Insbesonders war man bei Faktor VIII bisher der Ansicht, daß er, als einer der wärme-labilsten Gerinnungfaktoren, eine Erhitzung ohne Aktivitätsverlust bzw. Denaturierung überhaupt nicht übersteht. Ein weiterer Mangel der bekannten Verfahren, insbesondere bei Faktor VIII, besteht darin, diesen vom begleitenden Fibrinogen abzutrennen. Beide Proteine haben ähnliche physikalischchemische Eigenschaften und sind mit Methoden, die im Produktionsmaßstab angewendet werden können, nicht quantitativ voneinander zu trennen.

Es ist bekannt, daß das für die Instabilität der Blutgerinnungsfaktoren verantwortlich gemachte Prothrombin mit Adsorptionsmitteln oder Fallungsmitteln aus Plasmafraktionen entfernt werden kann. Dies gilt vor allem für die Faktor VIII enthaltende Fraktion. Es ist ebenfalls bekannt, daß der Faktor VIII zusammen mit Fibrinogen durch Glycin präzipitiert werden kann; daß Aminosäuren, insbesondere β-Alanin, fur eine partielle Abtrennung des Fibrinogens vom Faktor VIII besonders geeignet sind und schließlich, daß mineralische Adsorbenzien Verunreinigungen, die den Faktor VIII begleiten, zu binden vermögen.

Alle diese bekannten Einzelmaßnahmen und ihre Kombinationen konnten aber die Probleme der Instabilität der Blutgerinnungsfaktoren gegen Warme und ihren Abbau durch Proteasen nicht befriedigend lösen. Auch eine Ergänzung dieser Maßnahmen durch zusätzliche Fällungs- und Fraktionierungsschritte führte zu keinem durchgreifenden Erfolg. Insbesondere stellte sich heraus, daß das als Fällungsmittel verwendete β-Alanin mit

2

hoher Wahrscheinlichkeit für verschiedene Unverträglichkeitsreaktionen verantwortlich zu machen ist. Danach verbietet sich die Anwendung dieses Fällungsmittels bei Präparationen, die für die menschliche Therapie eingesetzt werden.

Aus Thromb. Diath. Haemorrh. 11, 64-74 (1964) ist bekannt, daß man Faktor VIII mit Hilfe von Aminosäuren präzipitieren kann. Danach ist es jedoch nicht möglich, Fibrinogen und Faktor VIII mit Hilfe einer Aminosäurefällung quantitativ voneinander zu trennen. Auch die reinsten Faktor VIII-Konzentrate, die zur Zeit auf dem Markt sind, enthalten noch bedeutende Mengen von Fibrinogen. Das gilt auch für die nach der Methode IV unter Verwendung von Polyethylenglykol hergestellten Konzentrate.

Überraschenderweise wurde nun gefunden, daß die den bisher bekannten Verfahren fur die Faktor VIII-Herstellung anhaftenden Mangel durch einfache Modifikationen behoben werden können und es durchaus möglich ist, ein fibrinogenfreies und hepatitissicheres Faktor VIII-Konzentrat herzustellen.

Es war weiterhin überraschend, daß die für Faktor VIII gefundene Modifikation auch bei Faktor XIII zu einer Verbesserung der Stabilisierung in wäßriger Lösung auch gegenüber dem Verfahren von Fukushima et al. führt, und daß darüberhinaus das Verfahren nicht nur auf Faktor VIII und Faktor XIII, sondern auch auf Faktor II, Antithrombin III und Plasminogen angewandt werden kann.

Es wurde schließlich gefunden, daß eine Erhitzung in Gegenwart einer bestimmten Stabilisatorkombination das resultierende Präparat sowohl hepatitissicher macht als auch das gerinnbare Fibrinogen praktisch quantitativ entfernt. Die vorliegende Erfindung kann deshalb sowohl als Verfahren zur Stabilisierung der Gerinnungsfaktoren in wäßriger Lösung gegen Wärme als auch als Verfahren zur Herstellung von fibrinogenfreien und hepatitissicheren Praparationen von Gerinnungsfaktoren angesehen werden.

Gegenstand der Erfindung ist ein Verfahren zur Herstellung eines hepatitissicheren und praktisch fibrinogenfreien Gerinnungs- oder Fibrinolysefaktors aus der Gruppe Faktor II, VIII, XIII, Antithrombin III sowie Plasminogen durch Erhitzen auf 60-70°C in Gegenwart von Glycin und einem Saccharid bei einem pH-Wert zwischen 6,5 und 8, dadurch gekennzeichnet, daß man die Erhitzung in Gegenwart von Saccharose in einer Konzentration von 40 bis 60 Gewichtsprozent und von Glycin in einer Konzentration von 1,0 bis 2,5 mol/l vornimmt.

Eine Übertragung von Hepatitis-Erregern ist auf diese Weise nach dem Stand des heutigen Wissens praktisch auszuschließen. Dies gilt vor allem in Verbindung mit Fällungsverfahren, bei denen der Wirkstoff im Überstand bleibt und die Hepatitisviren zusammen mit dem unlöslichen Niederschlag abgetrennt werden können. Eine praparation, die etwa 10 Stunden bei etwa 60°C in wüssriger Lösung gehalten wurde, gilt heute als praktisch hepatitissicher.

In einer bevorzugten Ausführungsform wird eine Faktor II, VIII, XIII, Antithrombin III oder Plasminogen enthaltende Lösung, vorzugsweise eine Plasma- oder Plazenta-Fraktion, mit 1 bis 2,5 mol/l Glycin und 40 bis 60 Gewichtsprozent Saccharose versetzt und 1 min bis 48 Stunden, vorzugsweise etwa 10 Stunden auf eine Temperatur von 60 bis 70°C erhitzt, wobei die kürzeste Zeit der höchsten Temperatur zuzuordnen ist und umgekehrt. Um eine maximale Ausbeute zu erreichen, muß der pH-Wert spezifisch auf die einzelnen in der Lösung vorhandenen Gerinnungsfaktoren abgestimmt werden. Allgemein ist ein pH-Wert in den Grenzen zwischen 6,5 und 8 einzuhalten.

Die Temperaturbehandlung kann in mehreren Schritten durchgeführt werden.

Im Hinblick auf die therapeutische Anwendung der Präparate kann die Lösung, welche den Gerinnungsfaktor enthält, mit gangigen biochemischen Verfahren weitergereinigt, gegebenenfalls mit protein-stabilisierenden Substanzen versetzt, sterilfiltriert und/oder lyophilisiert werden. Für die Konfektionierung verwendet man die für die Herstellung parenteral applizierbarer Präparate üblichen Maßnahmen.

Mit der Kombination von Glycin und Saccharose wird unter folgenden Bedingungen ein von gerinnbarem Fibrinogen freies Praparat erzielt: 1-2,5 mol/l Glycin und 40 bis 60 Gewichtsprozent Saccharose bei einer Behandlung von 15 bis 120 Minuten bei 35-40°C und danach 5 bis 15 Minuten bei 50 bis 60°C, bei einem pH-Wert von 6,8-7,5.

Für die Herstellung eines hepatitissicheren Präparates ist die Erhitzung über 10 bis 20 Stunden auf 60-70°C in Gegenwart von Saccharose in einer Konzentration von 40 bis 60 Gewichtsprozent und einer Glycin-Konzentration von 1,0-2,5 mol/l besonders vorteilhaft. Zweckmäßigerweise geht man von Fraktionen aus, in denen der Faktor, von dem eine Präparation hergestellt werden soll, angereichert ist, für Faktor VIII beispielsweise eine nach dem als Methode VI bekannten Verfahren von Cohn (J.Amer.Chem.Soc. (1946) 68, 459ff) erhaltene. Eine solche Fraktion wird aus Plasma mit Hilfe von 8 % Ethanol unter den von Cohn angegebenen Bedingungen ausgefällt und enthält neben verschiedenen Globulinen Fibrinogen und Faktor VIII. Gleicherweise ist als Ausgangsmaterial eine Kältefällung des Plasmas, das sogenannte Kryopräzipitat geeignet. Dieses ist erhältlich nach New England J. Med. 273, 1443-1447 (1965). Zur Gewinnung des Kryopräzipitats wird Prischplasma zunächst auf -30°C, danach auf +4°C gebracht und der dabei anfallende Rückstand gewonnen. Jede der genannten Fallungen enthält mehr oder weniger Prothrombin, das leicht aktivierbar ist und für den Aktivitätsverlust in Faktor VIII-Präparaten verantwortlich gemacht wird. Daher empfiehlt es sich, das Prothrombin vor Anwendung des Verfahrens gemäß der Erfindung zu entfernen, beispielsweise durch Adsorption an Aluminiumhydroxid oder Bariumsulfat, durch Fallung mit Acridinbasen oder durch Chromatographie an Ionenaustauscherharzen. Bevorzugt wird jedoch Aluminiumhydroxid in Gelsuspension verwendet.

Die Kontrollen von Maßnahmen zur Anreicherung und Reinigung von Faktor VIII sind dem Fachmann durch die Kenntnis von Bestimmungsmethoden für die betreffenden Substanzen geläufig. Unter Verwendung dieser Kontrollmethoden können die Verfahrensbedingungen unter dem Gesichtspunkt einer befriedigenden Ausbeute und einer befriedigenden Reinheit des Produktes gelenkt werden.

Sowohl für die Bestimmung des Fibrinogens als auch des Faktors VIII sind in der Literatur mehrere Verfahren beschrieben. Zweckmäßig wird die Bestimmung des Fibrinogens nach folgender Vorschrift durchgeführt:

In einem Zentrifugenröhrchen werden 1 ml der Fibrinogen enthaltenden Lösung mit 9 ml physiologischer NaCl-Lcsung verdünnt und mit 0,6 ml einer Lösung von 60 Einheiten Thrombin/ml zum Gerinnen gebracht. Nach 60-minütigem Stehenlassen bei 37°C wird der Ansatz 30 min bei ca. 150.000 RZB (relative Zentrifugalbeschleunigung nach DIN) in der Ultrazentrifuge geschleudert. Der Überstand wird dekantiert und das Sediment dreimal mit 50 ml physiologischer NaCl-Lösung auf einer Filternutsche gewaschen. Nach Trocknen des Sediments über Nacht im Vakuum wird über eine Bestimmung des Stickstoffgehaltes nach Kjeldahl auf den Eiweißanteil in mg umgerechnet, der als Fibrinogen angegeben wird. Diese Methode erlaubt auch die Bestimmung von Fibrinogen neben Faktor VIII.

Die Bestimmung des Faktors VIII erfolgt beispielsweise nach folgendem Verfahren:

1 Teil, z.B. 0,1 ml partielles Thromboplastin, z.B. hergestellt nach der deutschen Offenlegungsschrift 23 16 430 wird mit einem Teil Faktor VIII-Mangelplasma und einem Teil verdünntes Normalplasma vermischt. Diese Mischung wird 6 min bei 37°C gehalten. Nach Zusatz von einem Teil einer auf 37°C vorgewärmten 0,025 molaren Calciumchlorid-Lösung wird die Zeit bestimmt, die vom Zusatz der Calciumchlorid-Lösung bis zum Auftreten eines Gerinnsels verstreicht. Zur quantitativen Aussage wird die aus der Faktor VIII enthaltenden Lösung sich ergebende Gerinnungszeit unter Bezugnahme auf eine mit einer Normalplasma-Verdünnungsreihe erzielten Eichkurve abgelesen.

1 Internationale Einheit ( = 1 IE) Faktor VIII entspricht der Faktor VIII-Aktivität von 1 ml Normalplasma.

Für die Weiterreinigung eines durch die Aminosäure-Fällung von Fibrinogen befreiten Faktor VIII-Präparates kann nach verschiedenen Methoden verfahren werden, wobei immer die Aktivität des Faktors VIII im Vordergrund steht. Als besonders vorteilhaft hat sich erwiesen, die Aminosäure enthaltende Lösung der fibrinogenfreien Faktor VIII-Präparation mit einem Neutralsalz in einer Konzentration zu versetzen, welche Faktor VIII aus der wäßrigen Lösung verdrängt. Vorteilhaft wird die Fällung des Faktor VIII mit Natriumchlorid oder Kaliumchlorid vorgenommmen. Das Salz wird zweckmäßig als festes Salz zugegeben. Es kann auch in einer konzentrierten Lösung zugesetzt werden. Bei einer Endkonzentration von 10-20 g/100 ml des Salzes wird Faktor VIII präzipitiert. Der Rückstand kann durch Zentrifugation oder Filtration gewonnen werden.

Zum Erzielen eines hepatitissicheren Präparates wird der Rückstand in einer Lösung von 1-2,5 mol/l Glycin und 40-60 Gewichtsprozent Saccharose mindestens 10 Stunden auf 60-70°C erhitzt. Hierzu können auch Faktor VIII-Präparate eingesetzt werden, die nach einem anderen Verfahren als dem beschriebenen gewonnen wurden (Beispiel 2).

Eine hochtourige Zentrifugation kann häufig die Qualität der Präparation noch verbessern. Das Produkt ist danach sehr proteinarm. Spezifische Aktivitaten von 20-40 Einheiten Faktor VIII pro mg Eiweiß können erreicht werden. Für die Anwendung am Menschen muß die Präparation einer Sterilfiltration unterzogen werden.

Eine nach diesem Verfahren erhaltliche proteinarme Faktor VIII-Präparation, welche frei von gerinnbarem Fibrinogen und hepatitissicher ist, stellt im besonderen den Gegenstand der Erfindung dar.

Zur Erhöhung der Lagerstabilität ist es zweckmäßig, dem gereinigten Faktor VIII-Konzentrat proteinstabilisierende Substanzen, beispielsweise Proteine, Aminosäuren oder Kohlenhydrate, zuzusetzen. Schließlich kann das dieser Behandlung unterzogene Präparat in gefriergetrockneter Form zur Verfugung gestellt werden.

Das erfindungsgemäß erhältliche Produkt ist, in einer für die pharmazeutische Verabreichung geeigneten Lösung, ein Arzneimittel für die Behandlung von Koagulopathien und kann intravenös, vorteilhaft als Infusion, zur Therapie und Prophylaxe von durch Faktor VIII-Mangel bedingten Blutungen, beispielsweise bei Hämophilie-A-Kranken, eingesetzt werden.

Zur Gewinnung eines Faktor XIII-Konzentrats kann man von einer Fraktion humaner Plazenten ausgehen, wie sie beispielsweise nach der deutschen Patentschrift 20 63 069 erhalten wird. Dazu werden gefrorene humane Plazenten mit einer 0,5 g/100 ml NaCl enthaltenden Lösung extrahiert und der Faktor XIII aus dem gewebefreien Überstand mit einer Acridin-Base ausgefällt.

Nach Aufschließen des Acridin-Adduktes mit einer 2,5 g/100 ml NaCl-Lösung wird die Faktor XIII-haltige Lösung mit Cetylpyridiniumchlorid von sauren Begleitproteinen und Lipiden gereinigt und nach erneuter Behandlung mit der Acridin-Base, Aufschluß mit 2,5 g/100 ml NaCl-Lösung und Konzentrieren durch Fallen mit Ammoniumsulfat und Gelfiltration weitergereinigt. Die Faktor XIII-aktiven Fraktionen werden vereinigt und durch Druckfiltration oder Neutralsalzfällung konzentriert.

Die Aktivität des Faktor XIII wird mit einem Verdünnungstest bestimmt (Thromb. Diath. Haemorrh. (1970) 23, 455). Man macht sich dabei die unterschiedliche Löslichkeit des vernetzten und (mangels fibrinstabilisierenden Faktors) nicht vernetzten Fibrins in 1 g/100 ml Chloressigsaure zunutze. Mit steigenden Verdünnungen der zu bestimmenden Lösung werden zunächst aus Faktor XIII-freiem Fibrinogen mittels Thrombin Fibringerinsel erzeugt und mit 1 g/100 ml Chloressigsäure inkubiert. Danach wird diejenige Verdünnung, in der das Fibringerinsel gerade noch erhalten bleibt, bestimmt. Diese ist die Faktor XIII-Konzentration, die zur Vernetzung gerade ausreicht. In der nächsthöheren Verdünnung löst sich das Fibringerinsel auf.

Als Bezugssubstanz dient normales Mischplasma. Die in 1 ml enthaltene Faktor XIII-Aktivität ist als eine Einheit definiert. Die gesuchte fibrinstabilisierende Aktivität errechnet sich aus dem Verhältnis der Grenzwerte für die Verdünnung von Mischplasma und Testlösung.

Zur Herstellung eines hepatitissicheren Präparates wird das so charakterisierte Faktor XIII-Konzentrat mit Glycin und Saccharose versetzt und erhitzt unter Bedingungen, wie sie auch bei Faktor VIII beschrieben werden. Dabei ist es für die Ausbeute wichtig, den pH-Wert oberhalb von 6,8 zu halten. Unter diesen Bedingungen ist das erfindungsgemäße Verfahren dem in JP-A-5 359 018 beschriebenen überlegen.

Wie die folgende Tabelle zeigt, führt es zu wesentlich höheren Ausbeuten:

|  | Verfahren nach JP-A-5 359 018 | | Erfindungsgemäßes Verfahren | | |
|---|---|---|---|---|---|
| pH-Wert | 7,0 | 8,0 | 6,3 | 7,0 | 8,0 |
| Stabilisatoren: | | | | | |
| Saccharose (Gew.-%) | — | — | 50 | 50 | 50 |
| Glycin mol/l | — | 2,6 | 2 | 2 | 2 |
| Mannit % | 20 | — | — | — | — |
| Akt. vor Erhitzen E/ml | 133 | 100 | 50 | 83 | 83 |
| Akt. nach Erhitzen 10 Std./60°C E/ml | 0 | 33 | 17 | 50 | 50 |
| Ausbeute | 0 | 33 | 34 | 60 | 60 |

Für die Weiterreinigung des so behandelten Faktor XIII-Konzentrates kann wie beim Faktor VIII beschrieben verfahren werden. Zur Fällung des Faktor XIII mit Neutralsalz hat sich die Verwendung von Ammoniumsulfat in einer Endkonzentration von 10-40 g/100 ml als vorteilhaft erwiesen.

Für die Anwendung am Menschen wird das Produkt einer Sterilfiltration unterworfen. Zur Stabilisierung für die Gefriertrocknung wird die Faktor XIII-haltige Lösung mit Proteinen und Kohlehydraten, vorteilhaft mit Human-Albumin und Glucose versetzt.

Um zu einem hepatitissicheren Faktor II-Konzentrat zu gelangen, geht man von einer Fraktion aus, wie sie beispielsweise nach Thromb. Diath. Haemorrh. Suppl. 35, 61 (1969) erhalten wird. Dazu adsorbiert man Plasma, das aus einem mit 0,01 mol/l EDTA (Ethylendiamino-tetra-acetat) anticoagulierten Blut gewonnen wurde, mit Tri-Calciumphosphat und zentrifugiert ab. Dabei wird der Faktor II quantitativ an das Adsorbens gebunden und kann durch mehrfaches Eluieren mit 0 2 mol/l Tri Natriumcitrat wieder gewonnen werden. Die vereinigten Eluate werden durch kombinierte Alkohol- und Essigsäurefällungen bei Temperaturen von -8°C bis +4°C weiter gereinigt. Dabei wird der Faktor II gleichzeitig konzentriert.

Man nimmt das Konzentrat in einem geeigneten Puffer, vorteilhaft Kochsalz/Natriumcitrat auf und bestimmt die Aktivität des Faktors II.

Die Aktivitätsbestimmung ist dem Fachmann im allgemeinen bekannt. Sie kann zum Beispiel nach Dtsch.med.Wschr. 81, 516 (1956) erfolgen. Dazu wird ein Teil, z.B. 0,1 ml Faktor II-Mangelplasma und 1 Teil verdünntes Normalplasma vermischt. Diese Mischung wird 30 Sekunden bei +37°C gehalten. Danach setzt man 2 Teile calciumhaltiges Thromboplastin, z.B. hergestellt nach der deutschen Patentschrift 23 56 493, zu und bestimmt die Zeit, die bis zum Auftreten eines Gerinnsels verstreicht. Zur quantitativen Aussage wird die aus der Faktor II enthaltenden Lösung sich ergebende Gerinnungszeit unter Bezugnahme auf eine mit einer Normalplasma-Verdunnungsreihe erzielten Eichkurve abgelesen.

Eine Einheit Faktor II entspricht der Faktor II-Aktivität von 1 ml Normalplasma.

Zur Abtötung der Hepatitis-Viren werden der so charakterisierten Faktor II-Lösung Glycin und Saccharose zugesetzt und erhitzt, unter Bedingungen, wie sie bei Faktor VIII beschrieben werden.

Für die Weiterreinigung wird die erhitzte Faktor II-Lösung gegebenenfalls zentrifugiert und die aktive Komponente durch Ausfallen mit Neutralsalz oder Alkohol, vorteilhaft 15-40 Gewichtsprozent, bei einem pH-Wert von 5-6,5 konzentriert. Für die Anwendung am Menschen wird das Produkt einer Sterilfiltration unterworfen. Zur Stabilisierung bei der Gefriertrocknung kann der Zusatz von Antikoagulantien, wie beispielsweise Heparin, vorteilhaft sein.

Um ein von infektiösem Virus freies Antithrombin III-Präparat zu erhalten, kann man beispielsweise von einem Verfahren ausgehen, wie es in DE-AS 22 43 688 beschrieben wird. Danach wird Dextransulfat, Heparin oder Chondroitinsulfat, für sich alleine oder in Gegenwart von Agarose oder Lysin-Agarose, mit Bromcyan in alkalischem Medium durch Vernetzen unlöslich gemacht. Nach Äquilibrieren eines dieser Materialien in einer Chromatographiesäule mit geeignetem Puffer adsorbiert man damit Citrat-Plasma. Man wäscht das Gel frei von Plasma und eluiert die Antithrombin III-haltige Fraktion mit einem Puffer höherer Molarität. Gegebenenfalls kann die Präparation durch Gelfiltration über ein Molekularsieb weiter gereinigt werden. Nach Konzentrierung über eine Neutralsalzfällung, vorzugsweise Ammoniumsulfat, wird die Aktivität des Antithrombin III-Konzentrates bestimmt.

Dazu sind dem Fachmann sowohl immunologische als auch funktionelle Testmethoden bekannt. Vorzugsweise wird die in Laboratoriumsblatter 28, 65 (1978) beschriebene Methode verwendet: 2 Teile, z.B. 0,2 ml, Antithrombin III-Reagenz werden mit 2 Teilen verdünntem Normalplasma vermischt und 4 Minuten bei +37°C gehalten. 1 Teil dieses Ansatzes wird dann zu 3 Teilen einer standardisierten Rinderfibrinogenlösung gegeben und die Zeit gemessen, die bis zur Ausbildung eines Gerinnsels vorgeht.

Zur quantitativen Aussage wird die aus der Antithrombin III-enthaltenden Lösung sich ergebende Gerinnungszeit unter Bezugnahme auf eine mit einer Normalplasma-Verdünnungsreihe erzielten Eichkurve abgelesen. Eine Einheit Antithrombin III entspricht dessen Aktivität in 1 ml Normalplasma.

Zur Abtötung der Hepatitisviren wird das so charakterisierte Antithrombin III-Konzentrat mit Glycin und Saccharose in der für Faktor VIII beschriebenen Weise versetzt. Nach dem Erhitzen kann gegebenenfalls denaturiertes Protein durch Zentrifugation entfernt werden. Das Antithrombin III wird durch Druckdialyse oder Umfällen mit Neutralsalz, vorzugsweise mit 50-80 % w/v Ammoniumsulfat, konzentriert und weiter gereinigt. Zur Anwendung am Menschen wird es auf eine physiologische Salzkonzentration dialysiert und sterilfiltriert und kann zur längeren Lagerung auch lyophilisiert werden.

Ein hepatitissicheres Plasminogen-Präparat kann beispielsweise ausgehend von dem Verfahren nach DE-A-20 57 401 hergestellt werden. Dazu wird ein wasserunlösliches Copolymerisat hergestellt, in das eine Aminocarbonsaure mit ε-ständiger Aminogruppe einpolymerisiert ist.

Humanes Plasma wird mit diesem Adsorbens in Kontakt gebracht, wobei das Plasminogen am Adsorbens angereichert wird und durch Elution gewonnen werden kann.

Die Aktivität dieser Fraktion kann beispielsweise nach Med. Welt 26, 1996 (1975) bestimmt werden. Danach werden 2 Teile, z.B. 0,2 ml Plasminogenreagenz, mit 1 Teil verdünntem Normalplasma vermischt. Diese Mischung wird 3 Minuten bei +37°C gehalten. Danach werden 1,5 Internationale Einheiten Thrombin zugesetzt und die Zeit bestimmt, die bis zur Ausbildung eines Gerinnsels vorgeht. Zur quantitativen Aussage wird die aus der Plasminogen enthaltenden Lösung sich ergebende Gerinnungszeit unter Bezugnahme auf eine mit einer Normalplasma-Verdunnungsreihe erzielten Eichkurve abgelesen. Eine Einheit Plasminogen ist definiert als diejenige fibrinolytische Aktivität, die in 1 ml Normalplasma enthalten ist. Sie entspricht 5 000 Christensen-Einheiten.

Eine solchermaßen charakterisierte plasminogen-reiche Fraktion wird in einem pH-Bereich von 6,5 bis 8 mit Saccharose und Glycin versetzt und erhitzt unter Bedingungen, wie sie bei Faktor VIII beschrieben werden.

Nach Verdünnen der Lösung und gegebenenfalls Abzentrifugieren von entstandenem denaturiertem Protein wird das Plasminogen mit Neutralsalz, vorzugsweise mit Ammoniumsulfat, ausgefällt und kann nach Dialyse gegen eine isotonische Salzlösung sterilfiltriert und gefriergetrocknet werden.

Die Erfindung soll an den nachstehenden Beispielen naher erläutert werden:

**Beispiel 1**

Hepatitissicheres Faktor VIII-Konzentrat aus Humanplasma

3,9 l Plasma werden schnell auf -30°C abgekühlt und nach 24 Std. auf +4°C gebracht. Danach wird bei +4°C der Rückstand in einer Zentrifuge bei 2.000 x g (15 min) abgeschleudert und gewonnen. Das resultierende Kryopräzipitat (47 g) wird in 175 ml einer mit Natirumcitrat gepufferten Kochsalzlösung vom pH 7,0 bei 25°C aufgelöst. Man erhält eine 2,5 %ige Proteinlösung. Diese Proteinlösung wird mit 1/10 Volumenteil einer 25 %igen Al(OH)$_3$-Suspension (British Drug House, England) versetzt und 20 min gerührt. Die Aufschlämmung wird 30 min bei 3.000 x g zentrifugiert. Der Niederschlag wird verworfen. Danach versetzt man den Überstand mit festem Glycin bis zu einer Endkonzentration von 2,2 mol/l und erwärmt anschließend schnell auf 37°C. Nach 30 min bei dieser Temperatur wird der Ansatz ebenso schnell auf +5°C abgekühlt. Der dabei sich bildende Niederschlag wird in 30 min bei 3.000 x g abzentrifugiert und verworfen. Danach erwärmt man die überstehende Lösung abermals, diesmal auf 56°C, beläßt die Lösung 5 min bei dieser Temperatur, kühlt danach auf 20°C ab. Bei dieser Maßnahme fällt das restliche Fibrinogen zusammen mit Globulinen aus. Es wird bei 3.000 x g (30 min) abzentrifugiert. Die überstehende Faktor VIII-Lösung wird mit festem Kochsalz auf eine Endkonzentration von 15 % (w/v) gebracht und ca. 1 Std. bei einer Temperatur von 20°C belassen; dabei fällt die Faktor VIII-Aktivität aus. Sie wird 30 min bei 3.000 x g abzentrifugiert. Der Rückstand wird in 15 ml einer mit Natriumcitrat gepufferten Kochsalzlösung vom pH-Wert 7 aufgelöst. Nach Zentrifugieren und Klärfiltration wird das Filtrat mit 50 % w/w Saccharose und 2 mol/l Glycin versetzt und die viskose Lösung 10 Stunden auf 60°C erhitzt. Die erhitzte Lösung wird zur Herabsetzung der Viskosität mit dem gleichen Volumen eines Puffers verdünnt, der 2,2 einen pH-Wert von 6,8-7 besitzt. Aus dieser Lösung wird der Faktor VIII mit Kochsalzlösung ausgefällt; dafür wird eine Lösung mit 35 % (w/v) NaCl verwendet, die bei einem pH-Wert von 6,8-7 2,2 mol/l Glycin und 0,02 mol/l Citrat enthält. Von dieser gepufferten Kochsalzlösung wird soviel der Faktor VIII-haltigen Lösung zugesetzt, bis eine Endkonzentration von 15 % (w/v) erreicht ist. Der Niederschlag wird bei 3.000 x g abgeschleudert und in 12 ml Citrat-NaCl-Puffer mit 2 % Glycin und 0,5 % Human-Albumin aufgenommen. Von dieser Lösung wird die Faktor VIII-Aktivität bestimmt. Die Lösung enthielt 30 IE/ml - 1 IE entspricht der Aktivität von 1 ml frischem Citratmischplasma gesunder Spender.

**Beispiel 2**

Erhitzen eines nach Blood 28, 1011 (1966) hergestellten Faktor VIII-Präparates zur Inaktivierung von Hepatitisviren

Das Lyophilisat von 4 Abfüllungen Faktor VIII-Konzentrat mit jeweils ca. 250 IE wird bei 37°C in 40 ml einer wäßrigen Lösung aufgenommen, die 2,2 mol/l Glycin und 1 g/ml Saccharose enthält. Nach vollständigem Lösen des Inhalts wird die Abfüllung luftdicht verschlossen und im Wasserbad 10 Std. bei 60°C inkubiert.

Die zähflüssige Lösung wird nach Abkühlen bei Raumtemperatur 3 x 3 Std. gegen das 50fache Volumen Citratpuffer 0,02 mol/l, der 0,06 mol/l NaCl und 10 mg/ml Glycin enthält, dialysiert.

Das während des Erhitzens ausgefallene Protein, vorwiegend Fibrinogen, wird abzentrifugiert und die klare Lösung sterilfiltriert und lyophilisiert. Die Aktivität des Lyophilisats nach Lösen in 20 ml aqua dest. lag bei 26 IE/ml. Demegemäß konnte 4 Abfüllungen Faktor VIII-Konzentrat mit ca. 250 IE eine Abfüllung mit hepatitissicherem Material von ca. 500 IE hergestellt werden.

**Beispiel 3**

Herstellung eines hepatitissicheren Faktor XIII-Konzentrates aus Human-Plazenten

15 kg gefrorene Plazenten vom Menschen (diese Menge entspricht etwa 24 Plazenten) werden fein zerkleinert und mit 15 Liter einer 0,5 %igen Natriumchloridlösung verrührt. Das entstandene Gemisch wird auf 10°C erwärmt und zentrifugiert. Aus dem gewebefreien Überstand wird die fibrinstabilisierende Aktivität bei pH 6,0 mittels einer 3 %igen Diaminoethoxyacridinlactat-Lösung bis zu einer Konzentration von 8 % Diaminoethoxyacridinlactat, bezogen auf Eiweiß, gefällt und durch Zentrifugation isoliert. Das Zentrifugat wird in 9 Liter Wasser bei pH 7,0 suspendiert, gewaschen und wieder zentrifugiert. Der Rückstand wird in 8 Liter einer 2,5 %igen Natriumchloridlösung, die noch 0,125 % Ethylendiamintetraessigsäure (EDTA) enthält und auf pH 7,5 eingestellt wurde, aufgenommen, verrührt und nach 4 Stunden vom Unlöslichen separiert. Der Überstand wird mit Wasser auf 15 Liter aufgefüllt. Diese Lösung wird mit 0,3 Liter einer 3 %igen N-Cetylpyridiniumchlorid-Lösung bei pH 7,0 versetzt. Dadurch werden Begleitproteine und Mucopolysaccharide ausgefällt. Sie werden durch Zentrifugation entfernt. Zu der überstehenden Lösung werden 0,75 Liter einer 3 %igen Diaminoethoxyacridinlactat-Lösung gegeben, wodurch die fibrinstabilisierende Aktivität ausgefällt wird. Nach Abheben des Überstandes wird die Diaminoethoxyacridinlactat-Fällung mit 1 Liter einer 5 %igen Natriumchloridlösung, die 25 g EDTA enthält und einen pH-Wert von 7,5 besitzt, durch 2stündiges Rühren zerlegt. Das abgeschiedene Diaminoethoxyacridinlactat-Chlorid trennt man durch Filtration ab. Aus dem Filtrat wird der fibrinstabilisierende Faktor durch Zugabe von 25 % festem Ammonsulfat unter Rühren langsam ausgefällt.

Bis zu dieser Ammonsulfatfällung muß die Aufarbeitung der Plazenten möglichst rasch und bei Temperaturen zwischen 5 und 10°C erfolgen, um größere Aktivitätsverluste zu vermeiden. Der Niederschlag der Ammonsulfatfällung wird nach 4stündigem Stehen abzentrifugiert.

Zur Weiterreinigung werden 8 g der Ammonsulfatpaste mit 0,01 molarer EDTA-Lösung (pH 7,0) zu einem Brei verrührt und bei 4°C gegen einen 0,005 molaren Tris(hydroxymethyl)aminomethan-Salzsäure-(Tris-HCl)Puffer (pH 7,0) dialysiert, der 0,005 mol EDTA/l Puffer und 0,05 % Natriumazid enthält. Anschließend stellt man die Lösung auf pH 6,0 ein. Die dabei entstehende Fällung wird abgeschleudert und verworfen. Der Überstand wird auf pH 7,0 eingestellt und mittels Saulenchromatographie an vernetztem Dextran, erhältlich unter dem geschützten Warenzeichen Sephadex® gereinigt. Zu Elution dient eine 0,005 molare Tris-HCl-Pufferlösung (pH 7,0), die 0,005 mol EDTA/l Puffer und 0,1 % Natrumazid enthält. Die aktiven Fraktionen werden nach dem Durchlauf gesammelt, und der fibrinstabilisierende Faktor wird daraus mit Ammonsulfat gefällt, wozu 25 g pro 100 ml Eluat erforderlich sind. Der Niederschlag wird isoliert und in 0,005 molarem Tris-EDTA-Puffer pH 7,0 gelöst.

Nach 20stundiger Dialyse gegen 0,005 molaren Tris-EDTA-Puffer pH 7,0 wird der fibrinstabilisierende Faktor durch Einstellen des pH-Wertes auf 5,0 als Euglobulin ausgefallt. Den nach Zentrifugation entstandenen Rückstand löst man in 2 ml physiologischer Natriumchloridlösung, die 0,01 mol EDTA/l Lösung enthält und mit

# 0 018 561

0,2 n-Natriumhydroxid-Lösung auf pH 7,0 eingestellt worden war. In diese Lösung werden 2 g feste Saccharose eingebracht und nach vollständiger Lösung weiterhin 0,6 g Glycin in fester Form zugesetzt. Der pH-Wert wird auf 7,0 eingestellt. Diese Lösung wird 10 Std. auf 60°C erhitzt. Nach Erhitzen wird mit dem gleichen Volumen dest. Wasser verdünnt und der Faktor XIII durch Einrühren von 0,25 g Ammonsulfat pro 1 ml Lösung gefällt. Der Niederschlag wird durch Zentrifugation gewonnen und in 2 ml 0,85 % Kochsalzlösung, die 0,01 mol/l EDTA enthält, aufgenommen. Nach Zugabe von 0,1 ml 20 %igem Human-Albumin wird die Lösung durch ein bakteriendichtes Filter sterilfiltriert und nacheinander gegen physiologische Natriumchlorid-Lösung und gegen physiologische Natriumchlorid-Lösung mit 0,5 % Glukose dialysiert. Man bestimmt nun die fibrinstabilisierende Aktivität der Lösung im Vergleich mit Humanplasma und verdünnt mit glukosehaltiger Natriumchlorid-Lösung so weit, daß die Aktivität von 4 ml Lösung der Aktivität von 250 bis 300 ml Mischplasma entspricht. Pro 25 ml Verdünnungslösung setzt man außerdem noch 1 ml 20 %iges Human-Albumin zu. Nach Sterilfiltration wird in Portionen zu 4 ml abgefüllt und lyophilisiert.

Die gesamte aus 15 kg Plazenten gewonnene fibrinstabilisierende Aktivität liefert 12 Abfüllungen mit je 250 ml Plasmaaktivität. Um die gleiche Menge an fibrinvernetzender Aktivität aus Plasma zu isolieren, wären etwa 40 bis 60 Liter Blut notwendig, was etwa 80 bis 120 Blutspenden mit je 500 ml Blut entspräche.

## Beispiel 4

Herstellung eines hepatitissicheren Prothrombin-Konzentrates aus humanem EDTA-Plasma

Ausgehend von 400 Blutspenden zu 500 ml, mit jeweils 50 ml einer Lösung 0,07 %ig an EDTA-Natrium und 0,65 %ig an Kochsalz antikoaguliert, werden ca. 100 Liter Plasma gewonnen und innerhalb von 2 Tagen wie folgt weiterverarbeitet:

Das Plasma wird bei 20°C mit 800 g Tri-Calciumphosphat versetzt und 20 min gerührt. Danach trennt man das Adsorbens durch Zentrifugieren ab und versetzt den kompakten Niederschlag mit 5 l Tri-Natriumcitratlösung 0,18 mol/l. Man ruhrt 30 min bei 20°C und zentrifugiert erneut. Der Überstand (Eluat 1) wird dekantiert und der Rückstand erneut mit 2,5 l Tri-Natriumcitratlösung 30 min bei 20°C gerührt. Man zentrifugiert und dekantiert wieder, vereinigt den Überstand (Eluat 2) mit Eluat 1 und verwirft den Rückstand.

Zur Abtrennung von feinverteiltem Adsorbens zentrifugiert man die vereinigten Eluate 1 und 2 bei 2.000 x g. Den klaren Überstand (7,3 l) verdünnt man mit gleichem Volumen dest. Wasser und stellt den pH-Wert mit 2 N Essigsäure auf 6,8. Danach setzt man bei 8°C soviel Alkohol zu, daß eine Endkonzentration an Alkohol von 16 % v/v entsteht. Man schleudert den Niederschlag bei 0-4°C ab, stellt den Überstand mittels 2 N Eisessig auf pH 5,2 und bringt die Alkoholkonzentration bei 8°C auf 25 % v/v. Der Niederschlag, der hauptsächlich Faktor II enthält, wird in 1,2 l eines Puffers aufgenommen, der aus 9 Teilen 0,5 %iger Kochsalzlösung und 1 Teil 0,1 mol/l Tri-Natriumcitrat besteht. Die Lösung wird auf pH 6,8 eingestellt und mit 1 g pro ml Saccharose und danach 0,15 g pro ml Glycin versetzt. Im verschlossenen Gefäß wird diese Mischung 10 Std. bei 60°C erhitzt. Danach wird sie mit gleichen Volumen dest. Wasser verdünnt und denaturiertes Protein abzentrifugiert. Der Überstand wird erneut auf pH 5,2 gestellt und bei 8°C mit Alkohol auf eine Endkonzentration von 25 % v/v gebracht. Der Niederschlag wird zentrifugiert, der Überstand verworfen.

Den Niederschlag nimmt man in 200 ml des oben beschriebenen Kochsalz/Citrat-Puffers, pH 6,8 auf und dialysiert über Nacht gegen das 50fache des gleichen Puffers. Nach Zusatz von 10 USP-Einheiten Heparin pro ml wird die Lösung sterilfiltriert und lyophilisiert. Man erhält 10 Abfüllungen mit je 200 Einheiten Faktor II.

## Beispiel 5

Herstellung eines hepatitissicheren Antithrombin III-Konzentrates aus Human-Plasma

Zu einer Lösung von 100 ml Dextransulfat (20 mg/ml) werden 5 g BrCN zugegeben. Der pH-Wert wird dann mit Hilfe von 5 m NaOH auf 11,0 eingestellt, dieser pH-Wert min lang aufrechterhalten und dann mit 5 m HCl auf pH 8,5 zurückgestellt und das Gemisch unter Rühren über Nacht stehengelassen. Es bildet sich eine weiße, körnige, gelartige Paste. Die Paste wird in eine kleine Säule von 5 mm Durchmesser und 8 cm Länge gegeben und mit einem Puffer von pH-Wert 8,5, der 0,02 m Tris, 0,01 m Citrat und 0,15 m NaCl enthält, äquilibriert. 2 ml normales Plasma werden durch die Säule gegeben. Nach dem Durchgang durch die Säule hat das Plasma seine Koagulationsfähigkeit verloren. Die Säule wird zunächst mit dem Originalpuffer gewaschen und dann durch stufenweise Erhöhung der Salzkonzentration auf 1 m NaCl eluiert. Das Eluat wird mit 80 g Ammoniumsulfat pro 100 ml Lösung versetzt und 2 Std. gerührt. Der Niederschlag wird zentrifugiert und in 1 ml dest. Wasser aufgenommen. Danach werden 1 g Saccharose und nach deren vollständigem Lösen noch 0,3 g Glycin zugesetzt. Die Antithrombin III-haltige Lösung wird 10 Std. auf 60°C erhitzt.

Nach Zugabe von 4 ml dest. Wasser und 3 g Ammoniumsulfat wird nach 18 Std. Stehen bei 4°C der Antithrombin IIIhaltige Niederschlag abzentrifugiert und in 1 ml physiol. NaCl aufgenommen. Nach Dialyse gegen physiol. Kochsalzlösung enthält das Präparat 1,6 Einheiten Antithrombin III pro ml, was einer 80 %igen Ausbeute entspricht.

**0 018 561**

**Beispiel 6**

Herstellung eines hepatitissicheren Plasminogen-Konzentrates aus Human-Plasma

100 mg Copolymerisat aus Propylen und Maleinsäureanhydrid - wie in DE-A-20 57 401 näher beschrieben - werden in 0,15 mol/l Kaliumphosphatpuffer vom pH-Wert 7,5 in einem Gesamtvolumen vom 5 ml fein suspendiert. Dann setzt man unter Rühren 10 ml Hexamethylendiamin zu, die in 1 ml des oben angegebenen Phosphatpuffers gelöst werden. Anschließend werden 5 ml einer 2,5 %igen kaliumphosphatgepufferten Lysinlösung vom pH 7,5 dazu pipettiert und der Ansatz 24 Std. lang bei 4°C gerührt. Dann wird das unlösliche Vernetzungsprodukt durch Zentrifugation gewonnen, mit physiologischer Kochsalzlösung lysin-frei gewaschen, mit destilliertem Wasser gespült und lyophil getrocknet.

100 mg des lyophilisierten Vernetzungsproduktes werden nach Anteigen mit einigen ml Humanplasma in 70 ml Humanplasma suspendiert, der pH-Wert mit verdünnter Salzsäure auf 7,0 eingestellt und der Ansatz 30 min bei 37°C gerührt. Das Adsorbens wird dann in der Zentrifuge abgeschleudert und mit 0,15 m Natriumphosphatpuffer vom pH-Wert 6,4 so lange gewaschen, bis die Waschwasser proteinfrei sind. Die Elution des Plasminogens erfolgt mit 25 ml einer 0,1 mol/l Tris-(hydroxymethyl)-aminomethanlösung vom pH-Wert 10,0, die 0,05 mol/l Lysin enthält. Das Eluat wird mit normaler Salzsäure neutralisiert und unter Rühren gegen das gleiche Volumen gesättigter Ammonsulfatlcsung dialysiert. Die Fällung wird abzentrifugiert, in 2-3 ml 0,1 mol/l Dinatriumhydrogenphosphat-Lösung aufgenommen und zweimal 12 Std. gegen jeweils das 100fache Volumen der gleichen Lösung dialysiert. Das Dialysat wird mit 1 g Saccharose pro ml, danach mit 0,15 g Glycin pro ml Lösung versetzt und 10 Std. bei 60°C gehalten. Die Lösung wird danach mit dem gleichen Volumen an dest. Wasser verdünnt und dann gegen das 20 fache Volumen gesättigter Ammoniumsulfatlösung dialysiert. Der Niederschlag wird durch Zentrifugation gewonnen, in 3 ml 0,1 mol/l Dinatriumhydrogenphosphatlcsung aufgenommen und gegen 0,1 mol/l Natriumphosphatpuffer, pH 7,5 bis zum Elektrolytausgleich dialysiert.

Als Endprodukt erhält man 3 ml einer 0,31 %igen Plasminogenlösung mit 37 300 Christensen-Einheiten (Chr-E)/ml und 1 200 Chr-E/mg Protein. Das Ausgangsmaterial besitzt einen Plasminogentiter von 4 000 Chr-E/Ml und 53 Chr-E/mg Protein.

**Patentanspruch**

Verfahren zur Herstellung eines hepatitissicheren und praktisch fibrinogenfreien Gerinnungs- oder Fibrinolysefaktors aus der Gruppe Faktor II, VIII, XIII, Antithrombin III sowie Plasminogen durch Erhitzen auf 60-70°C in Gegenwart von Glycin und einem Saccharid bei einem pH-Wert zwischen 6,5 und 8, dadurch gekennzeichnet, daß man die Erhitzung in Gegenwart von Saccharose in einer Konzentration von 40 bis 60 Gewichtsprozent und von Glycin in einer Konzentration von 1,0 - 2,5 mol/l vornimmt.

**Claim**

Process for producing a "hepatitis-safe" and essentially fibrinogen-free coagulation or fibrinolysis factor of the group consisting of factors II, VIII, XIII, antithrombin III and plasminogen by heating to 60 to 80°C in the presence of glycin and a saccharide at a pH between 6.5 and 8, which comprises heating in the presence of sucrose in a concentration of 40 to 60 percent by weight and of glycin in a concentration of 1.0 to 2.5 mol/l.

**Revendication**

Procédé pour la préparation d'un facteur de coagulation ou de fibrinolyse, sans risque d'hépatite et pratiquement exempt de fibrinogène, du groupe des facteurs II, VIII, XIII, antithrombine III et plasminogéne, par chauffage à 60-70°C en présence de glycine et d'un saccharide, à une valeur de pH entre 6,5 et 8, caractérisé en ce que le chauffage est effectué en présence de saccharose à une concentration de 40 à 60% en poids et de glycine à une concentration de 1,0 à 2,5 mole/litre.